# EUROPEAN PATENT APPLICATION

(11) **EP 3 460 475 A1**
(43) Date of publication of application: **27.03.2019**
(21) Application number: 17799432.4
(22) Date of filing: 17.05.2017
(51) Int. Cl.: G01N 33/569, G01N 33/531, G01N 33/543, C07K 16/08

(54) **IMMUNOCHROMATOGRAPHIC ANALYSIS DEVICE FOR DETECTING ZIKA VIRUS**

(30) Priority: 17.05.2016 JP 2016098845
(71) Applicant: Tanaka Kikinzoku Kogyo K.K., Chiyoda-ku Tokyo 100-6422 (JP)
(72) Inventor: SUZUKI Keita, Hiratsuka-shi Kanagawa 254-0076 (JP); IWAMOTO Hisahiko, Hiratsuka-shi Kanagawa 254-0076 (JP)
(74) Representative: Hoffmann Eitle
(86) International application number: PCT/JP2017/018540
(87) International publication number: WO 2017/200008

(57) **Abstract**

The present invention relates to an immunochromatographic analysis device which makes it possible to diagnose an infection by a Zika virus simply and rapidly, and addresses the problem of providing an immunochromatographic analysis device which enables the reduction in the occurrence of a cross reaction with a virus that belongs to the family *Flaviviridae* and is different from a Zika virus to detect a Zika virus specifically. The present invention relates to an immunochromatographic analysis device for detecting a Zika virus, said device being provided with a sample addition unit, a labeling substance storage unit, a chromatograph medium unit having a detection section, and an absorption unit, wherein each of the labeling substance storage unit and the detection section contains an antibody capable of recognizing NS1 which is a nonstructural protein of a Zika virus and which is represented by SEQ ID NO: 1.

## Description

### Technical Field

The present invention relates to an immunochromatography analysis device for detecting Zika virus, an immunochromatography analysis kit and an immunochromatography analysis method.

### Background Art

Zika virus, which causes Zika virus infection, is a virus belonging to *Flaviviridae* (Flavivirus) together with dengue virus, Japanese encephalitis virus, West Nile virus and the like. It is known that Zika virus is distributed widely in vertebrates including human and spreads through vectors such as mosquitoes and ticks. It has been reported that Zika virus spreads by vertical transmission from mother to child, transmission through sexual contact, transmission through blood and the like.

When a person is infected with Zika virus, the person develops acute fever (so-called Zika fever) after a three- to twelve-day incubation period and develops various symptoms such as nonsuppurative conjunctivitis, headache, muscle pain and joint pain.

Effective medications or vaccines against Zika virus infection have not been developed so far. To prevent the spread of Zika virus infection, diagnosis of Zika virus infection at an early stage is desired.

Diagnosis of Zika virus infection has been made so far by detecting Zika virus RNA through a blood test, a urine test or a saliva test (Non Patent Literature 1). As a serological method, an antibody (IgM or IgG) to Zika virus that a patient with Zika virus infection has in the body is detected by ELISA or a fluorescent antibody technique (Non Patent Literature 2).

### Background Art Document

### Non Patent Literature

Non Patent Literature 1: Chen, LH; Hamer, DH (2 February 2016). "Zika Virus: Rapid Spread in the Western Hemisphere." Annals of Internal Medicine
Non Patent Literature 2: Hayes, Edward B. "Zika Virus Outside Africa". Emerging Infectious Diseases 15 (9): 1347-50

### Summary of Invention

### Technical Problem to be Solved by Invention

However, diagnosis of Zika virus infection through the detection of RNA requires special equipment, reagents and the like and thus is expensive. Also, the inspection requires time of half a day to around a day, and it takes a long time to obtain the inspection results.

In the method of detecting an antibody to Zika virus, diagnosis of Zika virus infection is made indirectly by detecting IgM or IgG that is produced in the human body after a certain lapse of time following the infection with Zika virus, rather than directly detecting Zika virus. Thus, the method is not suitable for diagnosing Zika virus infection at an early stage. Moreover, because the method is not for directly detecting Zika virus, it is difficult during diagnosis to distinguish from infections with, for example, dengue virus and West Nile virus, which belong to *Flaviviridae* like Zika virus. Accordingly, a method for diagnosing Zika virus infection from an early stage of infection by directly detecting Zika virus is desired.

Thus, an object of the invention is to provide an immunochromatography analysis device which enables simple and rapid diagnosis of Zika virus infection. Moreover, another object is to provide an immunochromatography analysis device which can find Zika virus infection at an early stage by directly detecting Zika virus itself and which can reduce a cross-reaction with a virus belonging to *Flaviviridae* other than Zika virus and specifically detect Zika virus.

### Solution to Problem

As a result of intensive study to solve the problems, the present inventors have found that the problems can be solved when antibodies which recognize Zika virus nonstructural protein NS1 are used in an immunochromatography analysis device, and the inventors have thus completed the invention.

Namely, the present invention is described as below.
1. An immunochromatography analysis device for detecting Zika virus in an analyte, including a sample addition part, a labeling substance retaining part, a chromatography medium part having a detection part and an absorption part,
   wherein the labeling substance retaining part and the detection part each contain an antibody recognizing Zika virus nonstructural protein NS1 of SEQ ID NO: 1.
2. The immunochromatography analysis device according to the above 1, wherein at least one of the labeling substance retaining part and the detection part contains an antibody recognizing at least one amino acid sequence of the three amino acid sequences of SEQ ID NOs: 2 to 4 which are present in the whole amino acid sequence of the nonstructural protein NS1.
3. The immunochromatography analysis device according to the above 2, wherein the labeling substance retaining part and the detection part each contain an antibody recognizing at least one amino acid sequence of the three amino acid sequences of SEQ ID NOs: 2 to 4 which are present in the whole amino acid sequence of the nonstructural protein NS1.
4. An immunochromatography analysis kit including the immunochromatography analysis device according to any one of the above 1 to 3 and an analyte dilution solution for diluting and developing an analyte.
5. An immunochromatography analysis method for detecting Zika virus in an analyte using the immunochromatography analysis kit according to the above 4 wherein the immunochromatography analysis method includes the following steps (1) to (4):
   (1) a step of adding an analyte-containing solution obtained by diluting the analyte with the analyte dilution solution as a sample to the sample addition part,
   (2) a step of causing the antibody recognizing Zika virus nonstructural protein NS1 held in the labeling substance retaining part to recognize Zika virus in the analyte,
   (3) a step of developing the analyte and the antibody as a mobile phase on the chromatography medium part, and
   (4) a step of detecting Zika virus in the developed mobile phase with the antibody recognizing Zika virus nonstructural protein NS1 contained in the detection part.

### Effects of Invention

In the invention, using antibodies that recognize nonstructural protein NS1 that Zika virus has in an immunochromatography analysis device, a cross-reaction with a virus or the like other than Zika virus can be inhibited, and Zika virus can be detected rapidly and specifically. In particular, using an antibody that recognizes at least one amino acid sequence of the three amino acid sequences of SEQ ID NOs: 2 to 4 that are present in the whole amino acid sequence of Zika virus NS1, Zika virus can be detected more specifically.

### Brief Description of Drawing

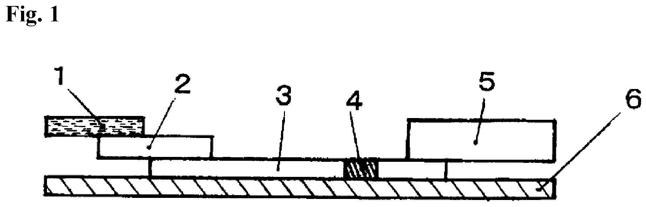

Fig. 1 is a cross section for explaining the structure of the immunochromatography analysis device of an embodiment of the invention.

### Description of Embodiments

Embodiments for carrying out the invention are explained below.

In this description, "immobilize" means that an antibody is arranged on a carrier such as a membrane in a manner that the antibody does not move, and "retain" means that an antibody is arranged in a manner that the antibody can move in a carrier such as a membrane or on the surface thereof.

In this description, that an antibody "recognizes" a specific protein means that the antibody binds to a part of the amino acid sequence that the protein has through the antigen-antibody reaction. Moreover, that an antibody "recognizes" a specific amino acid sequence means that the antibody binds to the whole or a part of the specific amino acid sequence through the antigen-antibody reaction.

### <Analyte>

The immunochromatography analysis device of the invention detects Zika virus in an analyte. The analyte that can be used for the invention is not particularly limited as long as the analyte may contain Zika virus. Specifically, the analyte is serum, plasma, whole blood, semen, spinal fluid or the like of an individual infected with Zika virus. Whole blood, serum and plasma are preferable in view of the rapid diagnosis.

### <Immunochromatography Analysis Device>

The immunochromatography analysis device for detecting Zika virus of the invention has a sample addition part to which a sample containing an analyte (also simply called a sample below) is applied, a labeling substance retaining part retaining a labeling substance, a chromatography medium part having a detection part for detecting Zika virus and an absorption part for absorbing a liquid which has passed through the detection part, and the device is characterized in that the labeling substance retaining part and the detection part each contain an antibody recognizing Zika virus nonstructural protein NS1.

### (Antibody)

The antibodies used for the immunochromatography analysis device of the invention are antibodies which recognize Zika virus nonstructural protein NS1 (also simply called NS1 below).

Zika virus NS1 is composed of the 352 amino acid residues of SEQ ID NO: 1. Although it has been suggested that Zika virus NS1 is a protein involving virus replication or the like as in other flaviviruses, the detailed functions and the structure are unknown.

In the invention, using antibodies recognizing especially NS1 of various proteins that Zika virus has, a cross-reaction with a virus or the like other than Zika virus can be inhibited, and Zika virus can be detected specifically. When the antibodies of the invention are used, for example, a cross-reaction with dengue virus, which belongs to *Flaviviridae* like Zika virus, or the like can be reduced. Specifically, as shown in the Examples described below, a cross-reaction with dengue virus nonstructural protein NS1 of SEQ ID NO: 6 is not caused.

In particular, it is preferable that at least one of the labeling substance retaining part and the detection part of the immunochromatography analysis device contains an antibody recognizing at least one amino acid sequence of the three amino acid sequences of SEQ ID NOs: 2 to 4 that are present in the whole amino acid sequence of NS1 of antibodies recognizing NS1. When an antibody recognizing at least one of the amino acid sequences is used, a cross-reaction with an antigen other than Zika virus can be reduced more, and Zika virus can be detected more specifically.

The amino acid sequence of SEQ ID NO: 2 (ENGVQLTVVV GSVKNPMWRG PQRLPVPVNE LPHGWKAWGK) corresponds to the amino acid sequence of from the 81st to 120th residues of the whole amino acid sequence of NS1, and the amino acid sequence of SEQ ID NO: 3 (SYFVRAAKTN NSFVVDGDTL KECPLEHRAW NSF) corresponds to the amino acid sequence of from the 121st to 153rd residues of the whole amino acid sequence of NS1. The amino acid sequence of SEQ ID NO: 4 (GP LSHHNTREGY RTQVKGPWHS EELEIRFEEC PGTKVYV) corresponds to the amino acid sequence of from the 249th to 287th residues of the whole amino acid sequence of NS1. Although detection is possible also using an antibody recognizing SEQ ID NO: 5 (CGTRGPSLRS TTASGRVIEE WCCRECTMPP), which is closer to the C-terminus and corresponds to the amino acid sequence of from the 291st to 320th residues of the whole amino acid sequence of NS1, it is speculated that the homologies to NS1 proteins of other viruses are high and that the specificity is thus not satisfactory.

Examples of the antibodies in the invention include natural antibodies such as polyclonal antibodies and monoclonal antibodies, chimeric antibodies, humanized antibodies or single-chain antibodies that can be produced using gene recombination, human antibodies that can be produced using a human antibody-producing transgenic animal or the like, antibodies produced by phage display and fragments thereof with binding capacity. Monoclonal antibodies are preferable in view of the sensitivity.

### (Production Method of Antibody)

Examples of the method for producing an antibody that recognizes Zika virus NS1 are explained below.

Regarding the kind of animal producing the antibody, for example, human, mouse, rat, rabbit, goat, horse and the like can be used. The immunoglobulin may be any of IgG, IgM, IgA, IgE and IgD.

In an embodiment, Zika virus NS1 peptide as the immunogen can be produced by a known general production method. That is, Zika virus NS1 peptide that is extracted and purified from Zika virus, Zika virus NS1 peptide that is obtained by expressing cloned gene of Zika virus NS1 in a host such as *Escherichia coli* by genetic engineering and extracting and purifying the peptide or a polypeptide which composes a part of Zika virus NS1 peptide can be used as the immunogen.

With respect to a monoclonal antibody, according to a general method, after hybridizing spleen cells of a mouse immunized with the immunogen and myeloma cells, a hybridoma that produces the target antibody is selected, and the monoclonal antibody produced by the hybridoma is obtained [for example, see the Kohler and Milstein's technique (Nature 256(1975)495-497)]. A polyclonal antibody is obtained by separating the target antibody from the antiserum obtained by immunizing an animal for production (for example, human, mouse, rat, rabbit, goat, horse or the like) with the immunogen according to a general method.

Screening to obtain the hybridoma clone that produces a monoclonal antibody can be conducted by culturing hybridomas for example in a microtiter plate and measuring the reactivities of the culture supernatants of the wells in which the growth is observed with the immunogen by enzyme immunoassay such as ELISA.

The hybridoma can be cultured using a medium (for example, DMEM containing 10% fetal bovine serum), and the supernatant of the culture solution obtained by centrifugation can be used as a monoclonal antibody solution. Also, ascites can be caused by injecting the hybridoma into the abdominal cavity of the origin animal, and the obtained ascites can be used as a monoclonal antibody solution. The monoclonal antibody is preferably isolated and/or purified.

In this manner, an antibody that recognizes Zika virus NS1 can be produced.

In this regard, commercial antibodies that recognize Zika virus NS1 can also be purchased and used. For example, clone 2801116 (Monoclonal Antibody To Zika Virus Ns1 Protein, Aalto Bio Reagent), clone 2901126 (Monoclonal Antibody To Zika Virus NS1 Protein, Aalto Bio Reagent) or the like can be purchased and used.

When antibodies that recognize Zika virus NS1 are produced in the above manner for example, antibodies that recognize at least one amino acid sequence of the three amino acid sequences of SEQ ID NOs: 2 to 4, of the antibodies that recognize Zika virus NS1, can be obtained and used by selecting hybridomas which produce antibodies exhibiting stronger reactivity with the three amino acid sequences of SEQ ID NOs: 2 to 4 from the hybridomas that produce antibodies recognizing Zika virus NS1 by ELISA test, western blotting or the like using peptide fragments corresponding to the three amino acid sequences of SEQ ID NOs: 2 to 4.

### (Immunochromatography Analysis Device)

Next, an embodiment of the immunochromatography analysis device of the invention is explained referring to the drawing.

In an embodiment, the immunochromatography analysis device of the invention is composed of a sample addition part (1), a labeling substance retaining part (2), a chromatography medium part (3), a detection part (4), an absorption part (5) and a backing sheet (6) as shown in Fig. 1.

The sample addition part (1) is a part in the immunochromatography analysis device to which a sample containing an analyte is applied. The sample addition part (1) can be composed of a porous sheet having the properties of rapidly absorbing the sample but allowing the sample to move rapidly. Examples of the porous sheet include cellulose filter paper, glass fibers, polyurethane, polyacetate, cellulose acetate, nylon, cotton cloth and the like.

The labeling substance retaining part (2) retains a labeled antibody labeled with a labeling substance described below (also simply called the labeled antibody below) and is a part in which the labeled antibody binds to the substance to be detected in the analyte. The labeled antibody is an antibody that recognizes Zika virus NS1, and the antibody binds to Zika virus NS1 in the analyte when the sample moves in the labeling substance retaining part (2).

For the labeling substance retaining part (2), a membrane of glass fibers, cellulose or the like is usually used.

The amount of the labeled antibody in the labeling substance retaining part (2) is usually 0.05 µg/device to 0.5 µg/device, preferably 0.05 µg/device to 0.25 µg/device, more preferably 0.07 µg/device to 0.1 µg/device. The amount of the labeled antibody per unit area of the labeling substance retaining part (2) is usually 0.05 µg/cm² to 1.0 µg/cm², preferably 0.1 µg/cm² to 0.8 µg/cm², more preferably 0.17 µg/cm² to 0.6 µg/cm².

An enzyme or the like is also generally used as the labeling substance for labeling an antibody in an immunochromatography analysis, but an insoluble carrier is preferably used as the labeling substance because the insoluble carrier is suitable for visually determining the presence of the substance to be detected. That is, in the invention, a labeled antibody which is labeled by sensitizing an antibody recognizing Zika virus NS1 by an insoluble carrier is preferably used as the antibody contained in the labeling substance retaining part (2). In this regard, as a method for sensitizing the antibody recognizing Zika virus NS1 by the insoluble carrier, a known method can be applied.

As the insoluble carrier used as the labeling substance, particles of a metal such as gold, silver or platinum, particles of a metal oxide such as iron oxide, particles of a nonmetal such as sulfur, latex particles of a synthetic polymer or other insoluble carriers can be used. As described above, the insoluble carrier is a labeling substance which is suitable for visually determining the presence of the substance to be detected and preferably has a color to make the visual determination easy. Metal particles and metal oxide particles themselves have peculiar natural colors according to the particle diameter, and the colors can be used as labels.

The insoluble carrier used as the labeling substance is especially preferably gold particles because gold particles are simple to detect and do not easily cohere and because nonspecific color development is unlikely to occur. The average particle diameter of the gold particles is, for example, 10 nm to 250 nm, preferably 35 nm to 120 nm. The average particle diameter can be calculated by measuring the projected area circle equivalent diameters of 100 particles at random using projected pictures taken with a transmission electron microscope (TEM: manufactured by JEOL Ltd., JEM-2010) and calculating from the average. The amount of the gold particles in the labeling substance retaining part is, per unit area of the labeling substance retaining part, usually 0.006 µg/cm² to 0.42 µg/cm², preferably 0.01 µg/cm² to 0.3 µg/cm², more preferably 0.01 µg/cm² to 0.2 µg/cm². This is because, by determining the amount in the range, the labeled particles can be developed while the particles are dispersed, and the recognition sites for the antibody are not inhibited, resulting in an increase in the sensitivity.

The chromatography medium part (3) is a part for development of chromatography. The chromatography medium part (3) is an inert membrane composed of a fine porous substance which causes a capillary phenomenon. For example, membranes made of nitrocellulose (also called nitrocellulose membranes below) and membranes made of cellulose acetate (also called cellulose acetate membranes below) are preferable, and nitrocellulose membranes are further preferable, because the membranes do not have the property of reacting with the detection reagent or the immobilizing reagent used for chromatography or with the substance to be detected or the like and because the effects of the invention are enhanced. In this regard, cellulose membranes, nylon membranes and porous plastic clothes (for example, polyethylene, polypropylene or the like) can also be used.

The nitrocellulose membranes may be any nitrocellulose membranes as long as the membranes mainly contain nitrocellulose, and membranes containing nitrocellulose as the main material, such as a pure product or a nitrocellulose-mixed product, can be used.

The nitrocellulose membranes can contain also a substance which further enhances the capillary phenomenon. The substance is preferably a substance which weakens the surface tension of the membrane surface and attains hydrophilicity. For example, a substance which has amphipathic action, like saccharides, derivatives of amino acids, fatty acid esters, various synthetic surfactants, alcohols or the like, and which does not affect the movement of the substance to be detected and does not affect the color development of the labeling substance is preferable.

The nitrocellulose membranes are porous and cause a capillary phenomenon. The indicator of the capillary phenomenon can be confirmed by measuring the speed of water absorption (water absorption time: capillary flow time). The speed of water absorption affects the detection sensitivity and the test time.

The form and the size of the chromatography medium part (3), which is typically any of the nitrocellulose membranes or the cellulose acetate membranes described above, are not particularly limited and may be any form or any size as long as they are appropriate for the actual operation and for the observation of the reaction results.

In order to further make the operation simpler, a support composed of plastic or the like is preferably provided on the back surface of the chromatography medium part (3). The properties and state of the support are not particularly limited, but when the measurement results are observed by a visual evaluation, the support is preferably a support having a color which is not similar to the color achieved by the labeling substance, and the support is usually preferably colorless or white.

In order to prevent the deterioration of the analysis accuracy due to nonspecific adsorption on the chromatography medium part (3), the chromatography medium part (3) can be subjected to blocking treatment by a known method according to the need. For the blocking treatment, in general, a protein such as bovine serum albumin, skim milk, casein or gelatin is preferably used. After the blocking treatment, the chromatography medium part (3) may be washed with one or a combination of two or more of surfactants such as Tween 20, Triton X-100 or SDS according to the need.

The detection part (4) is formed at any position on the chromatography medium part (3) and contains an antibody that recognizes Zika virus NS1. The antibody that recognizes Zika virus NS1 can be immobilized on the detection part (4) according to a general method.

In the detection part (4), Zika virus in the analyte that has passed through on the chromatography medium part as the mobile phase specifically binds in a manner that Zika virus is sandwiched between the antibody recognizing Zika virus NS1 that is immobilized on the detection part (4) and the antibody recognizing Zika virus NS1 to which the labeling substance is bound.

The amount of the antibody recognizing Zika virus NS1 contained in the detection part (4) is usually 0.1 µg/device to 3.0 µg/device, preferably 0.3 µg/device to 2.0 µg/device, more preferably 0.3 µg/device to 1.0 µg/device. The amount of the antibody recognizing Zika virus NS1 per unit area of the detection part (4) is usually 0.04 µg/cm² to 1.0 µg/cm², preferably 0.125 µg/cm² to 0.8 µg/cm², more preferably 0.125 µg/cm² to 0.42 µg/cm².

The absorption part (5) is provided at the end of the chromatography medium part (3) to absorb liquids such as the analyte and the development solution which have passed through the detection part (4). In the invention, for example, glass fibers, pulp, cellulose fibers or these nonwoven clothes to which a polymer such as acrylic polymers and a hydrophilic agent having an ethylene oxide group or the like have been added are used for the absorption part (5), and glass fibers are particularly preferable.

The backing sheet (6) is a base material. One surface thereof is adhesive because an adhesive is applied on the surface or an adhesive tape is attached, and the sample addition part (1), the labeling substance retaining part (2), the chromatography medium part (3), the detection part (4) and the absorption part (5) are partially or entirely closely adhered and provided on the adhesive surface. The base material is not particularly limited as long as the backing sheet (6) is not permeable or breathable with respect to the sample solution due to the adhesive.

The immunochromatography analysis device of the invention is usually subjected to drying treatment before being finished as a product. The drying temperature is, for example, 20°C to 50°C, and the drying time is 0.5 hours to 1 hour.

In the immunochromatography analysis device of the invention, at least one of the labeling substance retaining part and the detection part preferably contains an antibody recognizing at least one amino acid sequence of the three amino acid sequences of SEQ ID NOs: 2 to 4 that are present in the whole amino acid sequence of Zika virus NS1. When an antibody recognizing at least one of the amino acid sequences is used, a cross-reaction with another antigen can be reduced more, and Zika virus can be detected more specifically.

Here, even when only one of the labeling substance retaining part and the detection part contains an antibody recognizing at least one amino acid sequence of the three amino acid sequences of SEQ ID NOs: 2 to 4 that are present in the whole amino acid sequence of Zika virus NS1, the other one of the labeling substance retaining part and the detection part, which does not contain the antibody, may contain an antibody recognizing any amino acid sequence in the amino acid sequence of SEQ ID NO: 1.

It is more preferable that both of the labeling substance retaining part and the detection part each contain an antibody recognizing at least one amino acid sequence of the three amino acid sequences of SEQ ID NOs: 2 to 4 that are present in the whole amino acid sequence of Zika virus NS1.

Both of the antibodies contained in the labeling substance retaining part and the detection part may be antibodies recognizing at least one amino acid sequence of the three amino acid sequences of SEQ ID NOs: 2 to 4 that are present in the whole amino acid sequence of Zika virus NS1, and both of the antibodies contained in the labeling substance retaining part and the detection part may be antibodies that recognize the amino acid sequence of same SEQ ID NO. In this case, it is speculated that the antibody contained in the labeling substance retaining part and the antibody contained in the detection part bind to amino acid sequences at different sites in the amino acid sequence of same SEQ ID NO.

The immunochromatography analysis device of the invention can also be designed as explained below so that the device can detect Zika virus in the analyte and simultaneously detect an antibody (human IgM and/or human IgG) to Zika virus in the analyte.

In the immunochromatography analysis device of the invention, an antibody recognizing an antibody (human IgM and/or human IgG) in the analyte is separately contained in the labeling substance retaining part (2) in addition to the antibody recognizing Zika virus NS1.

In this case, a detection part which contains a peptide having the whole amino acid sequence of Zika virus NS1 of SEQ ID NO: 1 or the amino acid sequence of SEQ ID NO: 1 having deletion, substitution or addition of one to several amino acid residues, a peptide obtained by adding another protein or the like to such a peptide or the like is separately provided on the chromatography medium part (3) in addition to the detection part (4) described above. Preferably, a detection part which contains at least one peptide of the peptides having the amino acid sequences of SEQ ID NOs: 2 to 4 described above, any of the peptides having the amino acid sequences of SEQ ID NOs: 2 to 4 having deletion, substitution or addition of one to several amino acid residues or at least one peptide of peptides obtained by adding another protein or the like to the peptides is separately provided in addition to the detection part (4) described above.

When the immunochromatography analysis device has such a structure, an antibody to Zika virus in the analyte can also be detected.

This means that, when blood or the like collected from an individual infected with Zika virus is used as the analyte for example, an antibody (human IgM and/or human IgG) recognizing Zika virus NS1 contained in the analyte is recognized by the antibody recognizing human IgM and/or human IgG contained in the labeling substance retaining part (2), and the antibody recognizing human IgM and/or human IgG binds to the peptide contained in the detection part which is separately provided on the chromatography medium part (3) in addition to the detection part (4), which enables the detection of the antibody to Zika virus in the analyte.

As described above, when the immunochromatography analysis device is used, Zika virus in the analyte can be detected directly, and, at the same time, Zika virus infection can be diagnosed indirectly by detecting an antibody (human IgM and/or human IgG) to Zika virus in the analyte.

Because the ratio of Zika virus in the analyte to the antibody to Zika virus depends also on the time (days) from the infection with Zika virus or the like, the time (days) from the infection with Zika virus can be roughly estimated by simultaneously detecting the two.

In this regard, when the labeling substance retaining part contains both of the antibody recognizing Zika virus NS1 and the antibody recognizing an antibody to Zika virus as described above, it is preferable to select antibodies which do not react with each other or cohere as the antibodies. When the antibodies react with each other and cohere, the device may be designed in a manner that the antibodies do not come into contact with each other between the labeling substance retaining part and the detection part.

### <Immunochromatography Analysis Kit>

The immunochromatography analysis kit of the invention includes the immunochromatography analysis device and an analyte dilution solution for diluting and developing an analyte.

In the immunochromatography analysis kit of the invention, the analyte dilution solution can be used also as a development solution. Water is usually used as a solvent of the analyte dilution solution, and a buffer solution, a salt and a nonionic surfactant are contained. A kind or two or more kinds of a protein, a polymer compound (such as PVP), an ionic surfactant or a polyanion for, for example, promoting the antigen-antibody reaction or inhibiting a nonspecific reaction, an antibacterial agent, a chelating agent and the like may be further added.

When the analyte dilution solution is used as a development solution, the analyte and the development solution can be mixed in advance and then supplied/applied as the sample to the sample addition part for development, or the development solution may be supplied/applied to the sample addition part for development after supplying/adding the sample containing the analyte to the sample addition part in advance.

### <Immunochromatography Analysis Method>

The immunochromatography analysis method of the invention includes the following steps (1) to (4), and Zika virus contained in an analyte is detected using the immunochromatography analysis kit.
(1) A step of adding an analyte-containing solution obtained by diluting the analyte with the analyte dilution solution as a sample to the sample addition part
(2) A step of causing the antibody recognizing Zika virus nonstructural protein NS 1 held in the labeling substance retaining part to recognize Zika virus in the analyte
(3) A step of developing the analyte and the antibody as a mobile phase on the chromatography medium part
(4) A step of detecting Zika virus in the developed mobile phase with the antibody recognizing Zika virus nonstructural protein NS1 contained in the detection part

Each step is explained below.

### (1) A step of adding an analyte-containing solution obtained by diluting the analyte with the analyte dilution solution as a sample to the sample addition part

In the step (1), first, an analyte-containing solution is preferably obtained by adjusting or diluting the analyte with the analyte dilution solution to a concentration at which the analyte moves smoothly in the immunochromatography medium without deteriorating the measurement accuracy. Those described above can be used as the analyte dilution solution. Secondly, a certain amount (usually 0.1 ml to 2 ml) of the analyte-containing solution is applied as a sample onto the sample addition part (1). When the sample is applied to the sample addition part (1), the sample starts to move in the sample addition part (1).

The analyte used in the invention is an analyte that may contain Zika virus, which is the substance to be detected, as described above. Specific examples include serum, plasma, whole blood, semen, spinal fluid or the like of a patient infected with Zika virus, but the analyte is not limited to these examples.

### (2) A step of causing the antibody recognizing Zika virus nonstructural protein NS1 held in the labeling substance retaining part to recognize Zika virus in the analyte

The step (2) is a step for transferring the sample applied to the sample addition part in the step (1) to the labeling substance retaining part (2) and causing the antibody recognizing Zika virus NS1 to which the labeling substance is bound and which is held in the labeling substance retaining part to recognize Zika virus, which is the substance to be detected, in the analyte. Those described above can be used as the labeling substance.

### (3) A step of developing the analyte and the antibody as a mobile phase on the chromatography medium part

The step (3) is a step in which, after Zika virus, which is the substance to be detected, has been recognized by the antibody recognizing Zika virus NS1 to which the labeling substance is bound in the labeling substance retaining part in the step (2), the analyte and the antibody are caused to pass through on the chromatography medium part as a mobile phase.

### (4) A step of detecting Zika virus in the developed mobile phase with the antibody recognizing Zika virus nonstructural protein NS1 contained in the detection part

The step (4) is a step in which Zika virus in the analyte that has passed through on the chromatography medium part as the mobile phase specifically binds by a specific antigen-antibody binding reaction in a manner that Zika virus is sandwiched between the antibody recognizing Zika virus NS1 that is immobilized on the detection part and the antibody recognizing Zika virus NS1 to which the labeling substance has bound in the step (2), resulting in the coloration of the detection part.

When Zika virus, which is the substance to be detected, is absent, the labeling reagent dissolved in the water content of the sample does not cause the specific binding reaction even when the labeling reagent passes through the detection part on the chromatography medium part, and thus the detection part is not colored.

At the end, the water content in the analyte-containing solution moves to the absorption part (5).

### Examples

The invention is further explained below with Examples, but the invention is not limited to the following examples.

### Production Example 1 (Production of Antibodies)

Antibodies which recognize Zika virus NS1 were produced as follows.

First, a peptide having the amino acid sequence of Zika virus NS1 of SEQ ID NO: 1 was synthesized. A His-tag expression vector, pET302/NT-His, was cut with a restriction enzyme, *Eco*RI, then treated with alkaline phosphatase as dephosphorylation treatment and mixed with the peptide, and ligation reaction was caused using DNA Ligation Kit Ver. 2 (Takara Bio Inc.).

A recombinant NS1 plasmid to which the target gene had been incorporated was introduced into a recombinant protein expression host, *E. coli* BL(DE3)pLysS (Novagen). The transformed bacterium was cultured on an LB agar plate, and a colony obtained was cultured with LB liquid culture. The expression of the recombinant NS1 was induced by adding 1 mM IPTG (Takara Bio Inc.), and then *E*. *coli* was collected. The collected bacterium was suspended again in a solubilization buffer [0.5% Triron X-100 (sigma), 10 mM imidazole, 20 mM phosphate and 0.5 M NaCl (pH 7.4) (Amersham)] and solubilized by ultrasonic treatment, and the recombinant NS1 was purified using His trap Kit (Amersham). The purified protein was dialyzed using phosphate-buffered saline (referred to as PBS below), and the target recombinant NS1 was thus obtained.

Monoclonal antibodies to the recombinant NS1 were produced using the obtained recombinant NS1 as the antigen for immunization. The monoclonal antibodies were produced as follows according to a general method. The recombinant NS1 in an amount of 100 µg and an equivalent amount of Adjuvant Complete Freund (Difco) were mixed, and a mouse (BALB/c, five weeks old, Japan SLC, Inc.) was immunized three times. The spleen cells were used for cell fusion. Mouse myeloma cells, Sp2/0-Ag14 cells (Shulman *et al.,* 1978) were used for the cell fusion. A culture medium obtained by adding 0.3 mg/ml L-glutamine, 100 U/ml penicillin G potassium, 100 µg/ml streptomycin sulfate and 40 µg/ml Gentacin to Dulbecco's Modified Eagle Medium (Gibco) (DMEM) and further adding fetal bovine serum (JRH) at 10% was used for culturing the cells. The cells were fused by mixing the spleen cells of the immunized mouse and Sp2/0-Ag14 cells and adding polyethylene glycol solution (Sigma) thereto. The fused cells were cultured in HAT-DMEM [serum-containing DMEM containing 0.1 mM sodium hypoxantine, 0.4 µM aminopterin and 0.016 mM thymidine (Gibco)], and the production of antibodies in the culture supernatant was confirmed by enzyme-linked immunosorbent assay (ELISA). Antibody production-positive cells were cultured in HT-DMEM [serum-containing DMEM containing 0.1 mM sodium hypoxantine and 0.16 mM thymidine] and further cultured in serum-containing DMEM.

The cloned cells were injected into the abdominal cavities of mice (BALB/c, retired, Japan SLC, Inc.) to which 2,6,10,14-tetramethylpentadecane (Sigma) had been injected, and the ascites were collected. The ascites were subjected to a protein G column, and monoclonal antibodies were purified.

The monoclonal antibodies thus obtained were screened by direct ELISA method using a 96-well plate in which the recombinant NS1 was immobilized.

As a result, three types of antibody recognizing Zika virus NS1 were obtained. The three types of antibody are called antibodies No. 1 to No. 3 in the following explanation.

### Reference Example 1 (ELISA Test)

It was examined whether the antibodies recognizing Zika virus NS1 produced in Production Example 1 (antibodies No. 1 to No. 3) recognized at least one amino acid sequence of the three amino acid sequences of SEQ ID NOs: 2 to 4 by ELISA test. The peptides used for the ELISA test were three types of peptide having the amino acid sequences of SEQ ID NOs: 2 to 4 (peptides 1 to 3 shown below) and produced by solid-phase peptide synthesis, which is a general method for chemically synthesizing a peptide.
Peptide 1: ENGVQLTVVV GSVKNPMWRG PQRLPVPVNE LPHGWKAWGK (SEQ ID NO: 2)
Peptide 2: SYFVRAAKTN NSFVVDGDTL KECPLEHRAW NSF (SEQ ID NO: 3)
Peptide 3: GP LSHHNTREGY RTQVKGPWHS EELEIRFEEC PGTKVYV (SEQ ID NO: 4)

First, a mixture of peptides 1 to 3 (called a peptide mixture below) was immobilized at a concentration of 100 ng/mL in a Nunc Immuno modules (manufactured by Thermo Fisher Scientific, code 469949) ELISA 96-well plate.

As the primary antibodies, 100 µL of solutions of antibodies No. 1 to No. 3 produced above (1 µg/mL) were put into separate wells and incubated at 37°C for an hour. Then, the primary antibody solutions were removed, and the wells were washed three times with 300 µL of PBST (0.05% Tween 20 in PBS). Next, 100 µL of 1% BSA solution was added to the wells, and the plate was incubated at 7°C for 1.5 hours. Then, the BSA solution was removed, and the wells were washed three times with 300 µL of PBST (0.05% Tween 20 in PBS). The liquids remaining in the wells were removed by hitting the plate onto a paper towel.

As the secondary antibody, 100 µL of 1 mg/mL Anti Mouse IgG (H+L), Rabbit, IgG Whole, Peroxidase Conjugated (manufactured by Wako Pure Chemical Industries, Ltd., code 014-17611) was added to the wells, and the plate was incubated at 37°C for 1.5 hours. Then, the secondary antibody solution was removed, and the wells were washed three times with 300 µL of PBST (0.05% Tween 20 in PBS). The liquids remaining in the wells were removed by hitting the plate onto a paper towel.

Sure Blue Reserve TMB Microwell Peroxidase Substrate (1-Component) (manufactured by KPL, code 53-00-01) in an amount of 100 µL was added to the wells as a chromogenic substrate, and the reaction was advanced for 15 minutes. The reaction was stopped by adding 100 µL of 2N sulfuric acid. Then, the absorbances at 450 nm were measured using a microplate reader (manufactured by BIORAD). A mark + was given when the reaction was observed as compared to the blank, and a mark - was given when the reaction was not observed. The results are shown in Table 1.

### [Table 1]

**Table 1**

| Antibody | No. 1 | No. 2 | No. 3 |
|---|---|---|---|
| Peptide Mixture | + | + | - |

From the above results, it was found that antibodies No. 1 and No. 2 recognize at least one amino acid sequence of the three amino acid sequences of SEQ ID NOs: 2 to 4.

### <Example 1>

An immunochromatography analysis kit composed of an analyte dilution solution and an immunochromatography analysis device including a sample addition part (1), a labeling substance retaining part (2), a chromatography medium part (3) having a detection part (4) and an absorption part (5) was produced.

Antibody No. 1 produced above was used as the antibody contained in the labeling substance retaining part, and antibody No. 2 produced above was used as the antibody contained in the detection part. The details are explained below.

### (1) Production of Sample Addition Part

A nonwoven cloth composed of glass fibers (manufactured by Millipore Corporation: 300 mm × 30 mm) was used as the sample addition part.

### (2) Production of Labeling substance retaining part

To 0.5 ml of a colloidal gold suspension (manufactured by Tanaka Kikinzoku Kogyo K.K.: LC 40 nm), 0.1 ml of antibody No. 1 which had been diluted to a concentration of 0.05 mg/ml with a phosphate buffer (pH 7.4) was added, and the mixture was left to stand still at room temperature for 10 minutes.

Next, 0.1 ml of a phosphate buffer (pH 7.4) containing 1 mass% bovine serum albumin (BSA) was added, and the mixture was further left to stand still at room temperature for 10 minutes. Then, after stirring thoroughly, the mixture was centrifuged at 8000×g for 15 minutes, and the supernatant was removed. Then, 0.1 ml of a phosphate buffer (pH 7.4) containing 1 mass% BSA was added. A labeling substance solution was produced by the above procedures.

A solution obtained by adding 300 µL of a 10 mass% aqueous trehalose solution and 1.8 mL of distilled water to 300 µL of the labeling substance solution produced above was evenly applied to a 12 mm × 300 mm glass fiber pad (manufactured by Millipore Corporation) and then dried with a vacuum dryer, and the labeling substance retaining part was thus produced.

### (3) Production of Chromatography Medium Part and Detection Part

A sheet composed of nitrocellulose (manufactured by Millipore Corporation, product name: HF120, 300 mm × 25 mm) was used as a membrane.

Next, 150 µL of a solution obtained by diluting antibody No. 2 to a concentration of 1.0 mg/ml with a phosphate buffer (pH 7.4) containing 5 mass% isopropyl alcohol was applied to a detection part on the dried membrane in a line with a width of 1 mm using a dispenser for immunochromatography "XYZ3050" (manufactured by BIODOT) at an amount of 1 µL/mm (25 µL per sheet).

Moreover, to check whether the gold nanoparticle labeling reagent has been developed or not and to check the development speed, a solution obtained by diluting goat-derived antiserum having a broad affinity range with the gold nanoparticle labeling substance with a phosphate buffer (pH 7.4) was applied to a control part (a control line) in the downstream of the detection part. Then, by drying at 50°C for 30 minutes and drying at room temperature overnight, the chromatography medium part and the detection part were produced.

### (4) Production of Immunochromatography Analysis Device

Next, the sample addition part, the labeling substance retaining part, the chromatography medium part having the detection part and a nonwoven cloth made of glass fibers as an absorption part for absorbing the developed sample and the labeling substance were attached one by one to a base material composed of a backing sheet. Then, the obtained product was cut with a width of 5 mm with a cutter, and the immunochromatography analysis device was thus obtained. The length of the labeling substance retaining part in the direction of sample development was adjusted to 12 mm.

### (5) Preparation of Analyte Dilution Solution

A 50 mM HEPES buffer (pH 7.5) containing 1 mass% nonionic surfactant (a 1:1 mixture of NP-40 manufactured by Nacalai Tesque, Inc. and Nonidet MN-811 manufactured by NOF Corporation) was prepared and used as the analyte dilution solution for diluting an analyte.

### <Example 2>

The immunochromatography analysis kit of Example 2 was produced in the same manner as in Example 1 except that antibody No. 2 produced above was used as the antibody contained in the labeling substance retaining part and that antibody No. 1 produced above was used as the antibody contained in the detection part in Example 1.

### <Example 3>

The immunochromatography analysis kit of Example 3 was produced in the same manner as in Example 1 except that antibody No. 2 produced above was used as the antibody contained in the labeling substance retaining part in Example 1.

### <Example 4>

The immunochromatography analysis kit of Example 4 was produced in the same manner as in Example 2 except that antibody No. 3 produced above was used as the antibody contained in the labeling substance retaining part in Example 2.

### <Example 5>

The immunochromatography analysis kit of Example 5 was produced in the same manner as in Example 1 except that antibody No. 3 produced above was used as the antibody contained in the labeling substance retaining part in Example 1.

### Test Example 1 (Measurement Using Zika Virus NS1 Recombinant Antigen)

In this test, measurement was conducted using the immunochromatography analysis kits of Examples 1 to 5 produced above and using a Zika virus NS1 recombinant antigen as the analyte. Zika virus NS1 recombinant antigen (manufactured by MeridianLife Science) was used as the analyte, and analyte-containing solutions were prepared by diluting the analyte to a concentration of 5 ng/mL or 20 ng/mL with the analyte dilution solution and used as the positive analyte samples.

The prepared analyte-containing solutions each in an amount of 90 µL were applied to the sample addition parts of the immunochromatography analysis devices and developed, and the test lines were visually observed after 15 minutes. Any of the following marks was given: "+++" when the red test line developed a stronger color; "++" when the red line developed a strong color; "+" when the red line developed a color; "±" when the red line slightly developed a color; and "-" when the red line could not be visually observed. The results are shown in Table 2.

Negative analyte samples were produced using PBS and standard human serum (manufactured by Access Bio) instead of the positive analytes, and the same test was conducted. The results are shown in Table 2.

### [Table 2]

**Table 2**

| | | Example 1 | Example 2 | Example 3 | Example 4 | Example 5 |
|---|---|---|---|---|---|---|
| Labeling Substance Retaining part | | No. 1 | No. 2 | | No. 3 | |
| Detection Part | | No. 2 | No. 1 | No. 2 | No. 1 | No. 2 |
| Positive Analyte | Zika virus NS1 5 ng/mL | ++ | ++ | + | ± | - |
| | Zika virus NS1 20 ng/mL | +++ | ++ | ++ | + | + |
| Negative Analyte | PBS | - | - | - | - | - |
| | Standard human serum | - | - | - | - | - |

As a result, it was found that Zika virus in an analyte can be detected when the immunochromatography analysis devices of the invention using antibodies recognizing Zika virus NS1 are used. In particular, it was found that the immunochromatography analysis devices of Examples 1 to 3, in which the labeling substance retaining part and the detection part contain antibody No. 1 or No. 2 recognizing at least one amino acid sequence of the three amino acid sequences of SEQ ID NOs: 2 to 4, can detect Zika virus with higher sensitivity.

### Test Example 2 (Measurement Using Inactivated Zika Virus)

In this test, measurement was conducted using the immunochromatography analysis kits of Examples 1 to 4 produced above and using inactivated Zika virus as the analyte. Zika virus heat inactivated virus (manufactured by ZeptMetrix, concentration of original solution: TCID₅₀=1×10^{5.23} U/mL) was used as the analyte, and analyte-containing solutions were prepared by diluting the analyte to result in the original concentration, a 10-fold dilution, a 100-fold dilution and a 1000-fold dilution with the analyte dilution solution.

The measurement was conducted in the same manner as in Test Example 1 using the prepared analyte-containing solutions as the samples. The results are shown in Table 3.

### [Table 3]

**Table 3**

| | | Example 1 | Example 2 | Example 3 | Example 4 |
|---|---|---|---|---|---|
| | Labeling Substance Retaining part | No. 1 | No. 2 | | No. 3 |
| | Detection Part | No. 2 | No. 1 | No. 2 | No. 1 |
| Dilution Ratio | ×1 | +++ | +++ | ++ | ++ |
| | ×10 | +++ | +++ | + | + |
| | ×100 | +++ | ++ | + | - |
| | ×1000 | ++ | ++ | ± | - |

As a result, the immunochromatography analysis kits of Examples 1 to 4, which showed positive reaction when the analyte was the Zika virus NS1 recombinant antigen, showed positive reaction as well also when the analyte was the inactivated Zika virus.

In particular, it was found that the immunochromatography analysis kits of Examples 1 to 3, in which the labeling substance retaining part and the detection part contain antibody No. 1 or No. 2 recognizing at least one amino acid sequence of the three amino acid sequences of SEQ ID NOs: 2 to 4, can detect the inactivated Zika virus with higher sensitivity.

### Test Example 3 (Measurement Using Dengue Virus)

In this test, measurement was conducted using the immunochromatography analysis kits of Examples 1 to 4 produced above and using inactivated dengue virus as the analyte. Dengue virus type2 AbD (manufactured by serotec) was used as the analyte, and an analyte-containing solution was prepared by diluting the analyte to a concentration of 1×10⁷ pfu/mL with the analyte dilution solution.

The measurement was conducted in the same manner as in Test Example 1 using the prepared analyte-containing solution as the sample. The results are shown in Table 4.

### [Table 4]

**Table 4**

| | Example 1 | Example 2 | Example 3 | Example 4 |
|---|---|---|---|---|
| Labeling Substance Retaining part | No. 1 | No. 2 | | No. 3 |
| Detection Part | No. 2 | No. 1 | No. 2 | No. 1 |
| Dengue Virus | - | - | - | - |

As a result, it was found that the immunochromatography analysis kits of the invention do not cause a cross-reaction with dengue virus, which is highly homologous to Zika virus, and can specifically detect Zika virus.

From the results of the Examples above, it was found that Zika virus in an analyte can be detected when the immunochromatography analysis devices of the invention using antibodies recognizing Zika virus NS1 are used. In particular, it was found that immunochromatography analysis kits in which the labeling substance retaining part and the detection part each contain either of antibodies No. 1 and No. 2, which are antibodies recognizing at least one amino acid sequence of the three amino acid sequences of SEQ ID NOs: 2 to 4, can detect Zika virus with particularly high sensitivity.

Furthermore, it was found that Zika virus can be detected specifically without causing a cross-reaction with another virus which is highly homologous to Zika virus when the immunochromatography analysis devices of the invention using antibodies recognizing Zika virus NS1 are used.

Although the invention has been explained in detail using specific embodiments, it is obvious to one skilled in the art that various changes and modifications can be made without departing from the intension and the scope of the invention. The present application is based on a Japanese patent application filed on May 17, 2016 (patent application No. 2016-098845), which is hereby incorporated by reference in its entirety.

### Reference Signs List

1 Sample addition part
2 Labeling substance retaining part
3 Chromatography medium part
4 Detection part
5 Absorption part
6 Backing sheet

## Claims

1. An immunochromatography analysis device for detecting Zika virus in an analyte, including a sample addition part, a labeling substance retaining part, a chromatography medium part having a detection part and an absorption part,
wherein the labeling substance retaining part and the detection part each contain an antibody recognizing Zika virus nonstructural protein NS1 of SEQ ID NO: 1.

2. The immunochromatography analysis device according to claim 1, wherein at least one of the labeling substance retaining part and the detection part contains an antibody recognizing at least one amino acid sequence of the three amino acid sequences of SEQ ID NOs: 2 to 4 which are present in the whole amino acid sequence of the nonstructural protein NS1.

3. The immunochromatography analysis device according to claim 2, wherein the labeling substance retaining part and the detection part each contain an antibody recognizing at least one amino acid sequence of the three amino acid sequences of SEQ ID NOs: 2 to 4 which are present in the whole amino acid sequence of the nonstructural protein NS1.

4. An immunochromatography analysis kit including the immunochromatography analysis device according to any one of claims 1 to 3 and an analyte dilution solution for diluting and developing an analyte.

5. An immunochromatography analysis method for detecting Zika virus in an analyte using the immunochromatography analysis kit according to claim 4, wherein the immunochromatography analysis method includes the following steps (1) to (4):
(1) a step of adding an analyte-containing solution obtained by diluting the analyte with the analyte dilution solution as a sample to the sample addition part,
(2) a step of causing the antibody recognizing Zika virus nonstructural protein NS1 held in the labeling substance retaining part to recognize Zika virus in the analyte,
(3) a step of developing the analyte and the antibody as a mobile phase on the chromatography medium part, and
(4) a step of detecting Zika virus in the developed mobile phase with the antibody recognizing Zika virus nonstructural protein NS1 contained in the detection part.


SEQUENCE LISTING

<110>  TANAKA KIKINZOKU KOGYO K.K.

<120>  IMMUNOCHROMATOGRAPHY ANALYSIS APPARATUS FOR DETECTING ZIKA
       VIRUS

<130>  W522652

<150>  JP2016/098845
<151>  2016-5-17

<160>  6

<170>  PatentIn version 3.5

<210>  1
<211>  352
<212>  PRT
<213>  Zika Virus

<400>  1

Asp Val Gly Cys Ser Val Asp Phe Ser Lys Lys Glu Thr Arg Cys Gly
1               5                   10                  15

Thr Gly Val Phe Ile Tyr Asn Asp Val Glu Ala Trp Arg Asp Arg Tyr
            20                  25                  30

Lys Tyr His Pro Asp Ser Pro Arg Arg Leu Ala Ala Ala Val Lys Gln
        35                  40                  45

Ala Trp Glu Glu Gly Ile Cys Gly Ile Ser Ser Val Ser Arg Met Glu
    50                  55                  60

Asn Ile Met Trp Lys Ser Val Glu Gly Glu Leu Asn Ala Ile Leu Glu
65                  70                  75                  80

Glu Asn Gly Val Gln Leu Thr Val Val Val Gly Ser Val Lys Asn Pro
                85                  90                  95

Met Trp Arg Gly Pro Gln Arg Leu Pro Val Pro Val Asn Glu Leu Pro
            100                 105                 110

His Gly Trp Lys Ala Trp Gly Lys Ser Tyr Phe Val Arg Ala Ala Lys
        115                 120                 125

Thr Asn Asn Ser Phe Val Val Asp Gly Asp Thr Leu Lys Glu Cys Pro
    130                 135                 140

Leu Glu His Arg Ala Trp Asn Ser Phe Leu Val Glu Asp His Gly Phe
145                 150                 155                 160

Gly Val Phe His Thr Ser Val Trp Leu Lys Val Arg Glu Asp Tyr Ser
                165                 170                 175

Leu Glu Cys Asp Pro Ala Val Ile Gly Thr Ala Val Lys Gly Arg Glu
            180                 185                 190

Ala Ala His Ser Asp Leu Gly Tyr Trp Ile Glu Ser Glu Lys Asn Asp
        195                 200                 205

Thr Trp Arg Leu Lys Arg Ala His Leu Ile Glu Met Lys Thr Cys Glu
    210                 215                 220

Trp Pro Lys Ser His Thr Leu Trp Thr Asp Gly Val Glu Glu Ser Asp
225                 230                 235                 240

Leu Ile Ile Pro Lys Ser Leu Ala Gly Pro Leu Ser His His Asn Thr
                245                 250                 255

Arg Glu Gly Tyr Arg Thr Gln Val Lys Gly Pro Trp His Ser Glu Glu
            260                 265                 270

Leu Glu Ile Arg Phe Glu Glu Cys Pro Gly Thr Lys Val Tyr Val Glu
        275                 280                 285

Glu Thr Cys Gly Thr Arg Gly Pro Ser Leu Arg Ser Thr Thr Ala Ser
    290                 295                 300

Gly Arg Val Ile Glu Glu Trp Cys Cys Arg Glu Cys Thr Met Pro Pro
305                 310                 315                 320

Leu Ser Phe Arg Ala Lys Asp Gly Cys Trp Tyr Gly Met Glu Ile Arg
                325                 330                 335

Pro Arg Lys Glu Pro Glu Ser Asn Leu Val Arg Ser Met Val Thr Ala
            340                 345                 350

<210>  2
<211>  40
<212>  PRT
<213>  Zika Virus

<400>  2

Glu Asn Gly Val Gln Leu Thr Val Val Val Gly Ser Val Lys Asn Pro
1               5                   10                  15

Met Trp Arg Gly Pro Gln Arg Leu Pro Val Pro Val Asn Glu Leu Pro
            20                  25                  30

His Gly Trp Lys Ala Trp Gly Lys
        35                  40

<210>  3
<211>  33
<212>  PRT
<213>  Zika Virus

<400>  3

Ser Tyr Phe Val Arg Ala Ala Lys Thr Asn Asn Ser Phe Val Val Asp
1               5                   10                  15

Gly Asp Thr Leu Lys Glu Cys Pro Leu Glu His Arg Ala Trp Asn Ser
            20                  25                  30

Phe

<210>  4
<211>  39
<212>  PRT
<213>  Zika Virus

<400>  4

Gly Pro Leu Ser His His Asn Thr Arg Glu Gly Tyr Arg Thr Gln Val
1               5                   10                  15

Lys Gly Pro Trp His Ser Glu Glu Leu Glu Ile Arg Phe Glu Glu Cys
            20                  25                  30

Pro Gly Thr Lys Val Tyr Val
        35

<210>  5
<211>  30
<212>  PRT
<213>  Zika Virus

<400>  5

Cys Gly Thr Arg Gly Pro Ser Leu Arg Ser Thr Thr Ala Ser Gly Arg
1               5                   10                  15

Val Ile Glu Glu Trp Cys Cys Arg Glu Cys Thr Met Pro Pro
            20                  25                  30

<210>  6
<211>  380
<212>  PRT
<213>  Dengue Virus

<400>  6

Met Asn Ser Arg Ser Thr Ser Leu Ser Val Ser Leu Val Leu Val Gly
1               5                   10                  15

Val Val Thr Leu Tyr Leu Gly Val Met Val Gln Ala Asp Ser Gly Cys
            20                  25                  30

Val Val Ser Trp Lys Asn Lys Glu Leu Lys Cys Gly Ser Gly Ile Phe
        35                  40                  45

Ile Thr Asp Asn Val His Thr Trp Thr Glu Gln Tyr Lys Phe Gln Pro
    50                  55                  60

Glu Ser Pro Ser Lys Leu Ala Ser Ala Ile Gln Lys Ala His Glu Glu
65                  70                  75                  80

Gly Ile Cys Gly Ile Arg Ser Val Thr Arg Leu Glu Asn Leu Met Trp
                85                  90                  95

Lys Gln Ile Thr Pro Glu Leu Asn His Ile Leu Ser Glu Asn Glu Val
            100                 105                 110

Lys Leu Thr Ile Met Thr Gly Asp Ile Lys Gly Ile Met Gln Ala Gly
        115                 120                 125

Lys Arg Ser Leu Gln Pro Gln Pro Thr Glu Leu Lys Tyr Ser Trp Lys
    130                 135                 140

Thr Trp Gly Lys Ala Lys Met Leu Ser Thr Glu Ser His Asn Gln Thr
145                 150                 155                 160

Phe Leu Ile Asp Gly Pro Glu Thr Ala Glu Cys Pro Asn Thr Asn Arg
                165                 170                 175

Ala Trp Asn Ser Leu Glu Val Glu Asp Tyr Gly Phe Gly Val Phe Thr
            180                 185                 190

Thr Asn Ile Trp Leu Lys Leu Arg Glu Lys Gln Asp Val Phe Cys Asp
        195                 200                 205

Ser Lys Leu Met Ser Ala Ala Ile Lys Asp Asn Arg Ala Val His Ala
    210                 215                 220

Asp Met Gly Tyr Trp Ile Glu Ser Ala Leu Asn Asp Thr Trp Lys Ile
225                 230                 235                 240

Glu Lys Ala Ser Phe Ile Glu Val Lys Ser Cys His Trp Pro Lys Ser
                245                 250                 255

His Thr Leu Trp Ser Asn Gly Val Leu Glu Ser Glu Met Ile Ile Pro
            260                 265                 270

Lys Asn Phe Ala Gly Pro Val Ser Gln His Asn Tyr Arg Pro Gly Tyr
        275                 280                 285

His Thr Gln Thr Ala Gly Pro Trp His Leu Gly Lys Leu Glu Met Asp
    290                 295                 300

Phe Asp Phe Cys Glu Gly Thr Thr Val Val Val Thr Glu Asp Cys Gly
305                 310                 315                 320

Asn Arg Gly Pro Ser Leu Arg Thr Thr Thr Ala Ser Gly Lys Leu Ile
                325                 330                 335

Thr Glu Trp Cys Cys Arg Ser Cys Thr Leu Pro Pro Leu Arg Tyr Arg
            340                 345                 350

Gly Glu Asp Gly Cys Trp Tyr Gly Met Glu Ile Arg Pro Leu Lys Glu
        355                 360                 365

Lys Glu Glu Asn Leu Val Asn Ser Leu Val Thr Ala
    370                 375                 380